(19) Europäisches Patentamt
European Patent Office
Office européen des brevets

(11) Publication number: **0 156 601**
**B1**

(12) **EUROPEAN PATENT SPECIFICATION**

(45) Date of publication of patent specification: **23.12.87**

(51) Int. Cl.[4]: **C 07 C 143/11,** C 07 C 139/00

(21) Application number: **85301798.6**

(22) Date of filing: **14.03.85**

(54) **Preparation of sulphonates.**

(30) Priority: **16.03.84 GB 8406865**
**19.09.84 GB 8423740**
**02.11.84 GB 8427803**

(43) Date of publication of application:
**02.10.85 Bulletin 85/40**

(45) Publication of the grant of the patent:
**23.12.87 *Bulletin* 87/52**

(84) Designated Contracting States:
**BE DE FR GB IT NL SE**

(56) References cited:
**EP-A-0 116 929**
**GB-A-1 539 001**
**US-A-2 115 192**

(73) Proprietor: **BP Chemicals Limited**
**Belgrave House 76 Buckingham Palace Road**
**London, SW1W 0SU (GB)**

(72) Inventor: **Lidy, Werner August**
**BP Chemicals (Suisse) SA 243 Routes des Fayards**
**CH-1290 Versoix (CH)**

(74) Representative: **Krishnan, Suryanarayana Kalyana et al**
**BP INTERNATIONAL LIMITED Patents & Agreements Division Chertsey Road**
**Sunbury-on-Thames Middlesex TW16 7LN (GB)**

**EP 0 156 601 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the preparation of alkyl- or alkylphenol-polyoxyalkylene ethane sulphonates, hereafter termed "ethane sulphonates" for convenience, by reaction of the corresponding ethane halide, hereafter referred to as the "parent ethane halide", with a sulphite.

The ethane sulphonates referred to herein are known as surfactants and have the formula:

$$R{-}O{-}(C_xH_{2x}.\overset{\displaystyle R^1 \atop |}{C}HO)_m\,(C_2H_4O)_n.C_2H_4SO_3M \qquad (I)$$

wherein R is selected from a $C_1$—$C_{20}$ alkyl group and a phenyl group having at least one $C_1$—$C_{20}$ alkyl substituent,

$R^1$ is H or $CH_3$,

M is a cation,

x is an integer from 1 to 3

m has a value from 0 to 20 and n has an average value from 1 to 50 such that when $R^1$ is H and x is 1, the sum of m + n is from 1 to 50.

The reason for 'n' being an average value in practice is due to the fact that during synthesis of the parent ethane halide a range of such halides are formed having a Gaussian distribution of n values. Thus the average value of n in a product may be integers or fractions.

The alkyl group present in the grouping represented by R may be a straight or branched chain alkyl or isomeric mixtures of such groups and may be derived from simple alkyl halides or from olefin oligomers e.g. the isobutene oligomers. Where the group R represents an alkyl substituted phenyl group it may be a mono-, a di- or a tri-alkyl substituted phenyl group, such that at least one of the alkyl substituents has 1 to 20 carbon atoms.

Where $R^1$ is H, and x is 1, the products represented by formula (I) are simple ethoxy ethane sulphonates. The present invention however is not limited to ethoxy ethane sulphonates but embraces within its scope sulphonates which are prepared by reacting an alcohol or an alkyl phenol initially with an epoxide or a glycol different from ethylene oxide or ethylene glycol before reacting with the latter. For instance, an alkyl phenol may first be reacted with tetrahydrofuran (x = 3 and $R^1$ = H in formula (I)) and then ethoxylated to form a product containing the grouping

$$—(CH_2.CH_2.CH_2.CH_2.O)_m—(C_2H_4O)_n—C_2H_4SO_3M \qquad (II)$$

Alternatively the alkyl phenol may first be reacted with propylene oxide (x = 1 and $R^1$ = $CH_3$ in formula (I)) and then ethoxylated to form a product containing the grouping

$$(—CH_2.\overset{\displaystyle CH_3 \atop |}{C}H.O)_m—(C_2H_4O)_n—C_2H_4SO_3M \qquad (III)$$

The group M is a cation from the metal sulphite used for sulphonation of the parent ethane halide. The cation may be monovalent, divalent or multivalent, the number of sulphonic acid groups in the formula (I) above being appropriately modified depending upon the valency of the cation.

In the compounds represented by formula (I) it would be possible to make the ethane sulphonate hydrophobic or hydrophilic by varying the length of the alkyl group R and by controlling the average number of the polyoxyethylene groups, i.e. the value of 'n' in the molecule. For instance, the hydrophobic nature of the ethane sulphonates increases as the length of R increases and as the average value of 'n' decreases. In general hydrophobic ethane sulphonates have an R in which the alkyl groups have more than 12 carbon atoms and they have an 'n' (average) value of less than 5. Thus, in formula (I) if R = $C_{18}$, and n (average) = 3 the ethane sulphonate will be substantially hydrophobic.

On the other hand as the length of R decreases and the average value of 'n' increases, the ethane sulphonates become more and more hydrophilic. In general hydrophilic ethane sulphonates have an R in which the alkyl groups have less than 12 carbon atoms and they have an 'n' (average) value of more than 5. Thus in formula (I) if R = $C_9$ and n (average) = 7, the ethane sulphonate is substantially hydrophilic.

The preparation of alkane sulphonates, in particular ethane sulphonates, by reaction of an alkyl phenol ethoxy alkane chloride with aqueous sodium sulphite for several hours under superatmospheric pressure has been previously described in US Patent No. 2,115,192. This is the well known Strecker reaction. Such alkane chlorides containing long hydrocarbon chains and a low number of ethoxy units are insoluble in aqueous sodium sulphite and hence the speed of reaction is slow. Reaction rates or conversions may be improved by control of pH or addition of alkali metal iodides to the reaction. Generally continuous agitation is necessary to improve the rate of reaction. It has also been found that in such reactions the reaction mixture may become viscous and as the viscosity increases agitation of the reaction mixture becomes

difficult, heat transfer is reduced and close control of the reaction rates is virtually impossible. To speed up the reaction between the alkane chloride and an alkali metal or ammonium sulphite it has been proposed in UK Patent No. 1,499,618 to employ a quaternary ammonium salt, the presence of which enables the reaction to proceed under milder conditions.

It is an object of the present invention to improve the process for the production of ethane sulphonates by mitigating the problems of increased viscosity and low reaction rates.

Accordingly, the present invention is a process for the preparation of ethane sulphonates of formula (I) as hereinbefore defined comprising reacting one or more parent ethane halides with a metal sulphite in an aqueous medium characterised in that the aqueous medium comprises a preformed ethane sulphonate.

The parent ethane halide reactant is preferably an ethane chloride.

The metal sulphite used for sulphonation is suitably an alkali metal sulphite preferably sodium or potassium sulphite.

The composition of the aqueous sulphite solution is such that when using between 5 and 50% stoichiometric excess of sulphite over the ethane halide, the water to ethane halide weight ratio is from 1:2 to 5:2, preferably from 1:1 to 3:2.

The choice of preformed ethane sulphonate used in the reaction will depend upon the nature of the reactants and the reaction conditions. The preformed ethane sulphonate acts as a surfactant and facilitates mixing of the parent ethane halide, which is water insoluble and in the organic phase, with the aqueous sodium sulphite thereby increasing the rate of reaction. It is desirable to use a preformed ethane sulphonate which is at least as soluble in the aqueous reaction medium as the product ethane sulphonate being synthesised, preferably more soluble than the product ethane sulphonate.

The effective amount of the preformed ethane sulphonate employed is preferably from 2 to 10% w/w of the total reaction mixture.

The reaction of the present invention is suitably carried out at a temperature from 125 to 220°C.

Where the desired reaction product is a hydrophobic ethane sulphonate, the reaction mixture tends to become viscous as mentioned previously with the attendant problems of agitation and close control of the rate of reaction. In such a case it is desirable to add to the aqueous reaction medium an additional ethane halide which upon sulphonation is capable of forming a hydrophilic ethane sulphonate.

Thus according to a further embodiment the present invention is a process for the preparation of hydrophobic ethane sulphonates by reacting a parent ethane halide capable of forming a hydrophobic sulphonate upon sulphonation with a metal sulphite in an aqueous reaction medium characterised in that the aqueous medium comprises a preformed ethane sulphonate and the reaction mixture contains an additional ethane halide capable of forming a hydrophilic sulphonate upon sulphonation.

If the reaction is carried out in the presence of the additional ethane halide which is capable of forming a hydrophilic ethane sulphonate upon sulphonation, formation of highly viscous reaction mixtures can be avoided. In such a case the ratio of the parent ethane halide to the additional ethane halide is more than 3:1, preferably in the range 3:1 to 10:1.

Also in this case, since both types of ethane halides are reactive, both will undergo sulphonation under the reaction conditions and the reaction product will be a mixture of both hydrophobic and hydrophilic ethane sulphonates though predominating in the desired hydrophobic ethane sulphonate.

The sulphonation reaction of the present invention is suitably carried out in the presence of a diluent which is capable of aiding the separation of water and the reaction products from the reaction mixture. The diluents are suitably selected from $C_5$—$C_{12}$ mono- or poly-hydric aliphatic alcohols, especially linear alcohols. Of these n-hexanol is the most preferred. The presence of an alcohol such as n-hexanol facilitates the stripping of water from the reaction mixture because it forms an azeotrope therewith. Moreover, the azeotrope distillate when cooled to ambient temperature separates into water and alcohol (in this case n-hexanol) layers thereby facilitating recycle of the alcohol for further use without having to resort to uneconomic and wasteful separation procedures.

The amount of aliphatic mono- or polyhydric-alcohol employed as diluent will depend upon the reactants used. Generally, the aliphatic alcohol concentration used may vary from 2 to 10% w/w, preferably from 5 to 8% w/w of the total reaction mixture.

The process of the present invention is particularly suited for producing $C_1$—$C_{20}$ alkyl phenol-(ethoxy)$_{1-20}$ ethane sulphonates.

The present invention is most preferably carried out using a $C_{18}$-phenol-(ethoxy)$_3$-ethane halide, a $C_9$-phenol-(ethoxy)$_7$-ethane halide, sodium sulphite, a $C_{18}$-phenol-(ethoxy)$_3$-ethane sulphonate, n-hexanol and water as reactants to produce a mixed $C_9$/$C_{18}$-phenol (ethoxy)$_7$/(ethoxy)$_3$-ethane sulphonate.

The process of the present invention may be carried out batchwise or continuously.

The invention is illustrated by the following Examples and Comparative Tests. However, the scope of this invention includes equivalent embodiments, modifications and variations within the scope of this disclosure.

Example 1

Preparation of compound of formula II

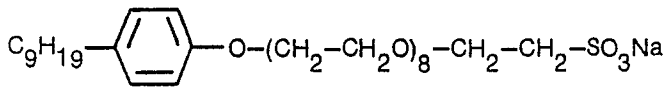

(IV)

(a) 1.1 mol of pure thionyl chloride were trickled into 1 mole of nonylphenol-(ethoxy)$_8$-ethanol starting at 30 to 35°C within 10—15 minutes. The reaction was exothermic and reached a temperature of about 50 to 60°C. Hydrogen chloride and sulphur dioxide were generated and were absorbed in a sodium hydroxide/water solution.

After addition of the thionyl chloride the liquid was heated to 90°C for about 2 hours to complete the reaction. To remove the remaining gaseous hydrogen chloride and sulphur dioxide a vacuum (30 mm Hg) was applied for another 2 hours at 90°C. The yield was 100% chloride determined by 13C NMR: disappearance of the C—OH peak at 62 ppm (tetramethyl silane), appearance of the C—Cl peak at 43 ppm.

(b) 58 g (0.46 mol) of pure sodium sulphite were dissolved in 240 g of water. 240 g (0.38 mol) of ethane chloride prepared in (a) and 4.8 g of a 90% nonyl phenol (ethoxy)$_8$-ethane sulphonate previously prepared were added and heated in a pressure tight reactor for 4 hours at 160°C. The initial emulsion turned into a solution during the reaction.

The aqueous sulphonate solution was treated with isopropanol to precipitate the inorganic salt byproducts which were then filtered off and the water was azeotropically distilled off. The refined, water free, product was a highly viscous liquid and was 90% pure (determined by 13C NMR C—$SO_3Na$ 50 ppm), the remaining 10% being the initial alcohol (C—OH 62 ppm).

Comparative Test 1

(a) The ethane chloride was made as in Example 1.

(b) The procedure of Example 1 (b) was followed except no final product was added.

After refining the product was only 60% pure, still containing non-reacted chloride and the alcohol.

Example 2

87 g (0.69 mol) of sodium sulphite were dissolved in 300 g of distilled water. 300 g (0.53 mol) of a 1:1 molar mixture of nonylphenol-(ethoxy)$_7$-ethane chloride and octadecylphenol-(ethoxy)$_3$-ethane chloride, together with 30 g of a 1:1 molar mixture of the sulphonates corresponding to these chlorides, were then added and the mixture heated in a pressure vessel for 8 hours at 180°C. The initial emulsion turned into a solution during the reaction without exhibiting a highly viscous intermediate stage. The torque measured on the agitator in the vessel, a measure of the viscosity of the reaction mixture, was between 12 and 17 N.cm throughout the reaction. The yield of the two sulphonates after 8 hours was 90% for the octadecylphenol-(ethoxy)$_3$-ethane sulphonate and 92% for the nonylphenol-(ethoxy)$_7$-ethane sulphonate as measured by Reverse Phase HPLC.

Comparative test 2

The method of Example 2 was used except that the reaction mixture comprised

(a) 91 g of sodium sulphite (0.72 mol) in 450 g water

(b) 300 g of octadecylphenol-(ethoxy)$_3$-ethane chloride (0.55 mol)

(c) 30 g of nonylphenol-(ethoxy)$_7$-ethane sulphonate.

In this test the initial emulsion turned into a solution during the reaction period but passed through a highly viscous intermediate stage which was difficult to agitate. The torque measured on the agitator during the reaction reached a maximum of 57 N.cm during the formation of highly viscous phase. Furthermore even after 9 hours reaction the yield of the product sulphonate was only 80%.

Example 3

91 g (0.72 mol) of pure sodium sulphite were dissolved in 450 grams of water. 300 grams (0.55 mol) of the parent $C_{18}$phenol (ethoxy)$_3$ ehtane chloride, 30 grams of a $C_9$-phenol-(ethoxy)$_7$-ethane sulphonate (90%) and 61 grams (7% on total mix) of hexanol were added and heated in a pressure tight reactor for 9 hours at 180°C. The initial emulsion turned into a solution during the reaction without exhibiting a pasty highly viscous stage. The torque measured on an agitator varied between 8 and 11 N cm. The final molar conversion of the product sulphonate was 80% measured by Reverse Phase high performance liquid chromatography (HPLC).

Comparative Test 3

The procedure of Example 3 was used but without hexanol addition. The initial emulsion turned into a solution during the reaction exhibiting a pasty, highly viscous stage. The torque measured on an agitator varied between 13 and 57 N.cm throughout the reaction.

## Claims

1. A process for the preparation of ethane sulphonates of the formula

$$R{-}O{-}(C_xH_{2x}.\overset{R^1}{\overset{|}{C}}HO)_m\,(C_2H_4O)_n.C_2H_4SO_3M \qquad (I)$$

wherein R is selected from a $C_1$—$C_{20}$ alkyl group and a phenyl group having at least one $C_1$—$C_{20}$ alkyl substituent,

$R^1$ is H or $CH_3$,

M is a cation,

x is an integer from 1 to 3

m has a value from 0 to 20 and n has an average value from 1 to 50 such that when $R^1$ is H and x is 1, the sum of m + n is from 1 to 50, the process comprising reacting one or more parent ethane halide with a sulphite of the cation M in an aqueous medium characterised in that the aqueous medium comprises a preformed ethane sulphonate.

2. A process according to claim 1 wherein the cation M is sodium or potassium.

3. A process according to claim 1 or 2 wherein the preformed ethane sulphonate is more soluble in the aqueous medium than the ethane sulphonate being prepared by the process.

4. A process according to anyone of the preceding claims wherein the reactant parent ethane halide used forms a hydrophobic ethane sulphonate upon sulphonation.

5. A process according to claim 4 wherein the reaction is carried out in the presence of an additional ethane halide capable of forming a hydrophilic ethane sulphonate upon sulphonation.

6. A process according to any one of the preceding claims wherein the group R in the ethane sulphonate of formula (I) represents an alkyl phenyl group.

7. A process according to any one of the preceding claims wherein the reaction is carried out in the presence of an aliphatic $C_5$—$C_{12}$ mono- or poly-hydric-alcohol as diluent.

8. A process according to claim 7 wherein the diluent is n-hexanol.

9. A process according to any one of the preceding claims wherein the reaction is carried out at a temperature from 125 to 220°C.

10. A process according to any one of the preceding claims wherein a $C_{18}$-phenol(ethoxy)$_3$-ethane halide is reacted with sodium sulphite in an aqueous medium in the presence of a $C_9$-phenol-(ethoxy)$_7$-ethane halide and a $C_{18}$-phenol-(ethoxy)$_3$-ethane sulphonate using n-hexanol as diluent to form a mixed $C_9$/$C_{18}$-phenol-(ethoxy)$_7$/(ethoxy)$_3$-ethane sulphonate.

## Patentansprüche

1. Ein Verfahren zur Herstellung von Ethansulfonaten der Formel

$$R{-}O{-}(C_xH_{2x}.\overset{R^1}{\overset{|}{C}}HO)_m\,(C_2H_4O)_n.C_2H_4SO_3M \qquad (I)$$

worin R ausgewählt ist aus einer $C_1$—$C_{20}$-Alkylgruppe und einer Phenylgruppe mit mindestens einem $C_1$—$C_{20}$-Alkylsubstituenten,

$R^1$ = H oder $CH_3$ ist,

M = ein Kation ist,

x = eine ganze Zahl von 1 bis 3 ist;

m einen Wert von 0 bis 20 hat und n einen durchschnittlichen Wert von 1 bis 50 hat, wobei die Summe von m + n = 1 bis 50 ist, wenn $R^1$ = H ist und x = 1 ist, bei welchem man ein oder mehrere Ausgangsethanhalogenid(e) mit einem Sulfit des Kations M in einem wässrigen Medium umsetzt, dadurch gekennzeichnet, daß das wässrige Medium ein vorgebildetes Ethansulfonat umfaßt.

2. Verfahren nach Anspruch 1, in welchem das Kation M Natrium oder Kalium ist.

3. Verfahren nach Anspruch 1 oder 2, in welchen das vorgebildete Ethansulfonat im wässrigen Medium stärker löslich ist als das durch das Verfahren hergestellte Ethansulfonat.

4. Verfahren nach irgendeinem der vorhergehenden Ansprüche, in welchem das als Reaktionsteilnehmer verwendete Ausgangsethanhalogenid nach Sulfonierung ein hydrophobes Ethansulfonat bildet.

5. Verfahren nach Anspruch 4, in welchem die Reaktion in Anwesenheit eines zusätzlichen Ethanhalogenids durchgeführt wird, das in der Lage ist, nach Sulfonierung ein hydrophiles Ethansulfonat zu bilden.

6. Verfahren nach Irgendeinem der vorhergehenden Ansprüche, in welchem die Gruppe R im Ethansulfonat der formel (I) eine Alkylphenylgruppe bedeutet.

7. Verfahren nach irgendeinem der vorhergehenden Ansprüche, in welchem die Reaktion in

Anwesenheit eines aliphatischen ein- oder mehrwertigen $C_5$—$C_{12}$-Alkohols als Verdünnungsmittel durchgeführt wird.

8. Verfahren nach Anspruch 7, in welchem das Verdünnungsmittel n-Hexanol ist.

9. Verfahren nach irgendeinem der vorhergehenden Ansprüche, in welchem die Reaktion bei einer Temperatur von 125 bis 220°C Durchgeführt wird.

10. Verfahren nach irgendeinem der vorhergehenden Ansprüche, in welchem ein $C_{18}$-Phenol(ethoxy)$_3$-ethanhalogenid mit Natriumsulfit in einem wässrigen Medium in Anwesenheit eines $C_9$—Phenol(ethoxy)$_7$-ethanhalogenids und eines $C_{18}$-Phenol(ethoxy)$_3$-ethansulfonates unter Verwendung von n-Hexanol als Verdünnungsmittel unter Bildung eines gemischten $C_9$/$C_{18}$-Phenol-(ethoxy)$_7$/(ethoxy)$_3$-ethansulfonates umgesetzt wird.

## Revendications

1. Procédé pour préparer des éthane sulfonates de formule

$$R—O—(C_xH_{2x}.\overset{\overset{R^1}{|}}{C}HO)_m\,(C_2H_4O)_n.C_2H_4SO_3M \qquad (I)$$

dans laquelle: R est choisi parmi un groupe alkyle en $C_1$ à $C_{20}$ et un groupe phényle comportant au moins un substituant alkyle en $C_1$ à $C_{20}$,

$R^1$ représente H ou $CH_3$,

M est un cation,

*x* est un nombre entier valant 1 à 3,

*m* vaut de 0 à 20 et *n* a une valeur moyenne allant de 1 à 50 de sorte que, lorsque $R^1$ représente H et *x* vaut 1, la somme de (m + n) vaut de 1 à 50.

le procédé comprenant la réaction d'un ou plusieurs halogénures d'éthanes générateurs avec un sulfite du cation M dans un milieu aqueux, et étant caractérisé en ce que le milieu aqueux comprend un éthane sulfonate préformé.

2. Procédé selon la revendication 1, dans lequel le cation M est le sodium ou le potassium.

3. Procédé selon la revendication 1 ou 2, dans lequel l'éthane sulfonate préformé est plus soluble dans le milieu aqueux que l'éthane sulfonate préparé par le procédé.

4. Procédé selon l'une quelconque des revedications précédentes, dans lequel l'halogénure d'éthane générateur que l'on fait réagir forme par sulfonation un éthane sulfonate hydrophobe.

5. Procédé selon la revendication 4, dans lequel on conduit la réaction en présence d'un halogénure d'éthane supplémentaire capable de former par sulfonation un éthane sulfonate hydrophile.

6. Procédé selon l'une quelconque des revendications précédentes, dans lequel le groupe R représente dans l'éthane sulfonate de formule (I) un groupe alkyl phényle.

7. Procédé selon l'une quelconque des revendications précédentes, dans lequel on conduit la réaction en présence d'un monoalcool ou polyalcool aliphatique en $C_5$ à $C_{12}$ comme diluant.

8. Procédé selon la revendication 7, dans lequel le diluant est le n-hexanol.

9. Procédé selon l'une quelconque des revendications précédentes, dans lequel on conduit la réaction à une température allant de 125 à 220°C.

10. Procédé selon l'une quelconque des revendications précédentes, dans lequel on fait réagir un halogénure de phénol (en $C_{18}$) (éthoxy)$_3$-éthane avec du sulfite de sodium en milieu aqueux, en présence d'un halogénure de phénol (en $C_9$) (éthoxy)$_7$-éthane et d'un phénol en $C_{18}$-(éthoxy)$_3$-éthane sulfonate, en utilisant du n-hexanol comme diluant, pour former un phénol en $C_9$/$C_{18}$-(éthoxy)$_7$/(éthoxy)$_3$-éthane sulfonate mixte.